# FASCICULE DE BREVET EUROPEEN

(11) **EP 0 944 624 B1**
(45) Date de publication et mention de la délivrance du brevet: **05.11.2003**
(21) Numéro de dépôt: 97913267.7
(22) Date de dépôt: 06.11.1997
(51) Int. Cl.: C07D 417/14, C07D 403/14, A61K 31/53

(54) **DERIVES INSOLUBLES DE S-TRIAZINE ET LEUR UTILISATION COMME FILTRES UV**
S-TRIAZINVERBINDUNGEN UND IHRE VERWENDUNG ALS UV-FILTER
INSOLUBLE S-TRIAZINE DERIVATIVES AND THEIR USE AS UV FILTERS

(30) Priorité: 13.12.1996 FR 9615368
(43) Date de publication de la demande: 29.09.1999
(73) Titulaire: L'OREAL, 75008 Paris (FR)
(72) Inventeur: RICHARD, Hervé, F-93420 Villepinte (FR); LEDUC, Madeleine-Résidence Les Chèvrefeuilles, F-75011 Paris (FR); LAGRANGE, Alain, F-77770 Coupvray (FR)
(74) Mandataire: Miszputen, Laurent
(86) Numéro de dépôt international: FR9701995
(87) Numéro de publication internationale: WO98025922

(56) Documents cités:
- EP-A- 0 165 608
- EP-A- 0 570 838
- EP-A- 0 704 444

## Description

La présente invention concerne de nouveaux dérivés insolubles de s-triazine portant des groupements benzotriazoles et/ou benzothiazoles, leur procédé de préparation et leurs utilisations sous forme particulaire en tant que filtres UV, notamment dans le domaine cosmétique.

La présente invention concerne également l'utilisation de ces nouveaux composés pour la protection de la peau et/ou des cheveux contre le rayonnement ultraviolet, ou pour la protection de toute autre matière sensible aux UV (verres minéraux ou organiques, plastiques ou autre).

On sait que les radiations lumineuses de longueurs d'onde comprises entre 280 nm et 400 nm permettent le brunissement de l'épiderme humain et que les rayons de longueurs d'onde comprises entre 280 nm et 320 nm, connus sous la dénomination d'UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 nm et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

Un grand nombre de composés ont déjà été proposés comme filtres solaires, sous la forme essentiellement de filtres organiques solubles ou de composés inorganiques insolubles. Ces filtres doivent pouvoir absorber ou bloquer les rayons nocifs du soleil tout en restant inoffensifs pour l'utilisateur.

Parmi les filtres organiques, on connaît les s-triazines solubles dans les huiles comme celles décrites dans les demandes de brevet EP0570838, les hydroxyphenyltriazines hydrosolubles telles que décrites dans le document EP0165608 ainsi que les dimères s-triazines hydrosolubles tels que décrites dans le document EP0704444.

A cet égard, et afin de limiter les éventuels risques d'allergie sur la peau engendrés par les filtres organiques du fait de leur solubilité, on utilise de plus en plus, pour filtrer les rayons UV, des pigments minéraux tels que l'oxyde de zinc ou encore l'oxyde de titane.
Cependant, ces pigments minéraux présentent l'inconvénient d'être sensibles au rayonnement solaire (phénomène connu sous le nom de photobleuissement). Par ailleurs, à quantités équivalentes, ces pigments minéraux sont moins efficaces dans la protection UV que les filtres organiques susmentionnés.

Or, à la suite d'importantes recherches menées dans le domaine de la photoprotection évoqué ci-dessus, la Demanderesse a maintenant découvert de nouveaux filtres UV, insolubles, non minéraux, capables d'absorber à la fois dans l'UV-A et i'UV-B, et qui présentent l'avantage de cumuler à la fois des propriétés de diffusion, puisque ce sont des pigments organiques solides, et d'absorption.

Cette découverte est à la base de l'invention.

Ainsi, selon la présente invention, il est maintenant proposé de nouveaux composés répondant à la formule suivante (I) : 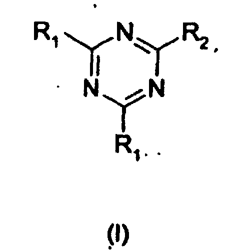 dans laquelle les symboles R₁, identiques ou différents, sont les radicaux de formules (II) ou (III) suivantes : 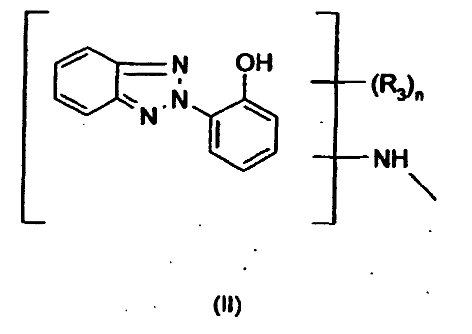 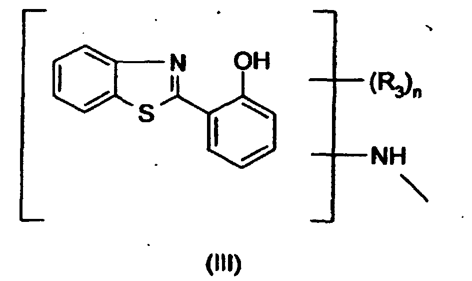
- R₂ est un halogène, N(R₄)₂, OR₅ ou un groupement R₁,
- R₃ ,identiques ou différents, sont des radicaux alkyle en C₁-C₈ linéaires ou ramifiés, des radicaux alkoxy en C₁-C₃ étant entendu que, dans ce dernier cas, deux R₃ adjacents d'un même noyau aromatique peuvent former ensemble un groupement alkylidène dioxy dans lequel le groupe alkylidène contient de 1 à 2 atomes de carbone, OH, NHCOCH₃ ou NH₂,
- R₄, identiques ou différents, désignent un atome d' hydrogène ou un radical alkyle en C₁-C₆ linéaire ou ramifié, deux R₄ pouvant former ensemble un cycle de 4 ou 5 atomes de carbone,
- n est 0, 1, 2, 3,
- R₅ est un hydrogène ou un radical alkyle en C₁-C₆.

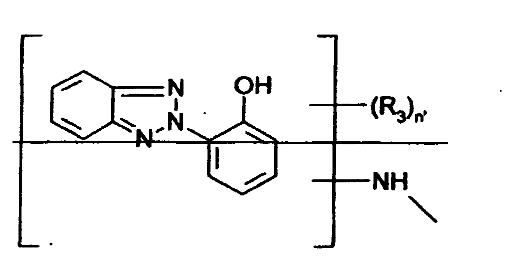

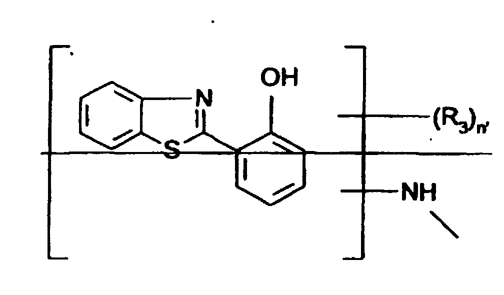

Ces nouveaux composés s-triaziniques insolubles peuvent être utilisés comme filtres solaires pour la peau humaine et les cheveux ainsi que comme agents protecteurs de la lumière dans l'industrie des plastiques.

Les dérivés de l'invention sont des composés insolubles et capables d'absorber simultanément dans l'UV-A et dans l'UV-B. Les radicaux de formules (II) et (III) sont des motifs filtrants qui absorbent généralement le rayonnement UV-A ; les nouveaux composés insolubles conformes à la présente invention, substitués par ces radicaux, présentent l'avantage inattendu et surprenant d'absorber à la fois dans l'UV-A et dans l'UV-B.

Par ailleurs, outre leurs propriétés filtrantes et dispersantes, ces nouveaux dérivés présentent une bonne stabilité chimique et photochimique. Du fait de leur insolubilité, ils présentent peu de risques de pénétration dans l'épiderme. Ces composés sont donc tout indiqués pour la préparation de compositions destinées à la protection solaire de la peau et des cheveux.

Par composés insolubles ou substantiellement insolubles, on entend, au sens de la présente invention, des composés dont la solubilité dans l'eau est inférieure à 0,1 % en poids, dont la solubilité dans l'huile de vaseline est inférieure à 1% en poids, et enfin, dont la solubilité dans un mélange d'esters de triglycérides tel que le « Miglyol 812 » commercialisé par la société Dynamit Nobel est inférieure à 2%, également en poids.

La présente invention a également pour objet un procédé de préparation des composés de formule (I) définis ci-avant consistant à faire réagir un composé de formule (IV) ci-dessous avec les dérivés de formules R₁H et R₂H selon le schéma réactionnel ci-dessous : 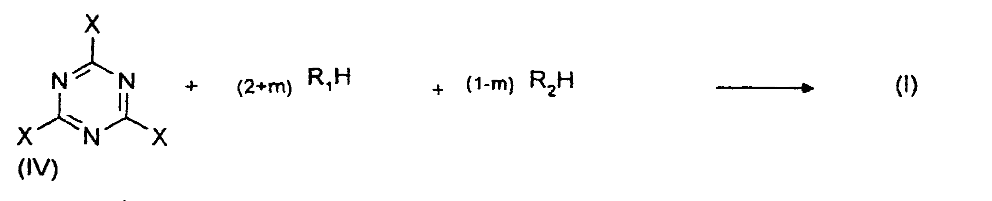 où :
- R₁ et R₂ répondent aux définitions données pour la formule (I) ci-dessus,
- X représente un halogène, en particulier le chlore ou le brome,
- m est 0 ou 1.

Les composés R₁H peuvent être préparés selon des méthodes connues telles que décrites dans les brevets DE 2,128,005, EP 221,630, FR 1,324,897 ou dans l'article de T.Konstantinova et al, Polymer Degradation and Stability, 43, 187 (1994).

Ainsi, comme dérivés aminés de benzotriazole, on peut citer :
- le 2-(2-hydroxy-5-aminophenyl)-5-methoxybenzotriazole décrit dans le document EP 221,630,
- le 2-(2-hydroxy-4-aminophenyl)benzotriazole, le 2-(2-hydroxyphenyl)-5-aminobenzotriazole et le 2-(2-hydroxy-5-methylphenyl)-5-aminobenzotriazole décrits dans les documents US 3,159,646 et GB 1,346,764,
- le 2-(2-hydroxy-5-aminophenyl)benzotriazole décrit dans le document J.Belusa et al, Chem.Zvesti,
- le 2-(2-hydroxy-5-aminophenyl)-5-chlorobenzotriazole et le 2-(2-hydroxy-4-aminophenyl)-5-chlorobénzotriazole décrits dans le document H.S.Freeman et al, Dyes and Pigments, 20, 171 (1992),
- le 2-(2-hydroxy-3-amino-5-methylphenyl)benzotriazole, le 2-(2-hydroxy-3-amino-5-ter-butylphenyl)benzotriazole et le 2-(2-hydroxy-3-amino-5-ter-octylphenyl)-benzotriazole qui peuvent être préparés selon des méthodes décrites dans les documents DE 2,128,005 et GB 1,346,764.

Comme dérivé aminé de benzothiazole, on peut citer par exemple le 2-(para-amino phényl)-6-methylbenzothiazole décrit dans le document US 2,334,348.

Les réactions ci-dessus peuvent être effectuées éventuellement en présence d'un solvant (toluène, xylène ou acétone/eau).

Parmi les composés conformes à l'invention, on peut citer plus particulièrement :
- la 2,4,6-tris[(3'-benzotriazol-2-yl-2'-hydroxy-5'-methyl) phenylamino]-s-triazine,
- 2,4,6-tris[(3'-benzotriazol-2-yl-2'-hydroxy-5'-ter-octyl) phénylamino]-s-triazine.

Ces nouveaux dérivés de s-triazine insolubles ou substantiellement insolubles peuvent être amenés sous une forme particulaire convenable par tout moyen ad-hoc tel que notamment broyage à sec ou en milieu solvant, tamisage, atomisation, micronisation, pulvérisation. Ils peuvent ensuite être utilisés comme pigments pour la protection solaire de la peau humaine et des cheveux. Ils peuvent également être utilisés comme agents protecteurs de la lumière dans l'industrie des plastiques, du verre (emballage, verres optiques, notamment pour lunetterie) et autres.

La présente invention a également pour objet une composition destinée à protéger une matière sensible au rayonnement ultraviolet, en particulier au rayonnement solaire, comprenant une quantité efficace d'au moins un composé de formule (I).

Plus particulièrement, lorsque la matière sensible à protéger est la peau et/ou les cheveux, cette composition se présente sous la forme d'une composition cosmétique comprenant, dans un support cosmétiquement acceptable, une quantité efficace d'au moins un composé de formule (I).

De préférence, les composés selon l'invention sont utilisés, dans les compositions cosmétiques conformes à l'invention, sous forme particulaire, la taille moyenne des particules étant inférieure à 20 µm.

Le ou les composés de formule (I) peuvent être présents dans la composition cosmétique selon l'invention dans des proportions comprises entre 0,1 et 20% en poids, par rapport au poids total de la composition, de préférence entre 0,1 et 15%.

La composition cosmétique de l'invention peut être utilisée comme composition protectrice de l'épiderme humain ou des cheveux ou comme composition antisolaire.

Les compositions selon l'invention peuvent bien entendu contenir un ou plusieurs filtres solaires complémentaires actifs dans l'UVA et/ou l'UVB (absorbeurs), hydrophiles ou lipophiles, autres que ceux de la présente invention. Ces filtres complémentaires peuvent être notamment choisis parmi les dérivés cinnamiques, les dérivés salicyliques, les dérivés du camphre, les dérivés de triazine autres que les composés conformes à la présente invention, les dérivés de la benzophénone, les dérivés du dibenzoylméthane, les dérivés de β,β-diphénylacrylate, les dérivés de l'acide p-aminobenzoïque, les polymères filtres et silicones filtres décrits dans la demande WO-93/04665.
D'autres exemples de filtres organiques sont donnés dans la demande de brevet EP-A 0 487 404.

Les compositions selon l'invention peuvent également contenir des agents de bronzage et/ou de brunissage artificiels de la peau (agents autobronzants), tels que par exemple de la dihydroxyacétone (DHA).

Les compositions selon l'invention peuvent encore contenir des pigments ou bien encore des nanopigments (taille moyenne des particules primaires généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A- 0 518 772 et EP-A- 0 518 773.

Les compositions de l'invention peuvent comprendre en outre des adjuvants cosmétiques classiques notamment choisis parmi les corps gras, les solvants organiques, les épaississants ioniques ou non ioniques, les adoucissants, les antioxydants, les opacifiants, les stabilisants, les émollients, les silicones , les α-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les poly-α-oléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose, l'hydroxypropylméthyl cellulose ou encore l'hydroxyéthylcellulose.

Bien entendu, l'homme de l'art veillera à choisir le ou les éventuels composés complémentaires cités ci-dessus et/ou leurs quantités de manière telle que les propriétés avantageuses attachées intrinsèquement au composé conforme à l'invention ne soient pas, ou substantiellement pas, altérées par la ou les adjonctions envisagées.

Lorsque la composition selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de stick, de mousse aérosol ou de spray.

Lorsque la composition selon l'invention est utilisée pour la protection des cheveux, elle peut se présenter sous forme de shampooing, de lotion, de gel, d'émulsion, de dispersion vésiculaire non ionique, de laque pour cheveux et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Les compositions de l'invention peuvent être préparées selon les techniques bien connues de l'homme de l'art, en particulier celles destinées à la préparation d'émulsions de type huile-dans-eau ou eau-dans-huile.
Lorsque la matière sensible à protéger est un verre organique et/ou minéral ou une matière plastique, les compositions selon l'invention peuvent se présenter sous la forme d'un vernis que l'on applique sur ladite matière sensible afin de la protéger du rayonnement ultraviolet.

La présente invention a encore pour objet l'utilisation d'au moins un composé de formule (I) dans des, ou pour la fabrication de, compositions destinées à protéger des matières sensibles au rayonnement ultraviolet, en particulier au rayonnement solaire.

En particulier, elle a pour objet l'utilisation d'au moins un composé de formule (I) dans des, ou pour la fabrication de, compositions cosmétiques pour la protection de la peau et/ou des cheveux contre le rayonnement ultraviolet, en particulier le rayonnement solaire.

La présente invention a également pour objet l'utilisation d'au moins un composé de formule (I) dans des, ou pour la fabrication de, vernis destinés à protéger des verres organiques et/ou minéraux ou des matières plastiques du rayonnement ultraviolet, en particulier du rayonnement solaire.

Les composés de l'invention peuvent également être incorporés directement dans des matières plastiques, ou dans d'autres matières sensibles au rayonnement ultraviolet, en vue de protéger ces dernières contre ledit rayonnement.

La présente invention a ainsi également pour objet un procédé cosmétique de protection de la peau et/ou les cheveux contre le rayonnement ultraviolet et/ou solaire, caractérisé par le fait qu'il consiste à appliquer sur la peau et/ou les cheveux une quantité efficace d'une composition cosmétique telle que définie ci-dessus.

Dans une autre forme de réalisation de l'invention, le procédé selon l'invention consiste à incorporer dans une matière plastique une quantité efficace d'un composé de formule (I) ou d'une composition contenant au moins un composé de formule (I) afin de protéger ladite matière plastique contre le rayonnement ultraviolet, en particulier contre le rayonnement solaire.

Ainsi, un objet de l'invention est une composition de matière plastique protégée par un tel procédé.

Dans une autre forme de réalisation de l'invention, le procédé selon l'invention consiste à appliquer une quantité efficace dudit composé ou de ladite composition à la surface d'un verre minéral ou organique.

Ainsi, finalement, un dernier objet de l'invention est une composition verrière protégée par ce procédé.

Des exemples concrets, mais nullement limitatifs, illustrant l'invention, vont maintenant être donnés.

### EXEMPLE 1 :

### Préparation de la 2,4,6-tris[(3'-benzotriazol-2-yl-2'-hydroxy-5'-methyl) phenylamino]-s-triazine :

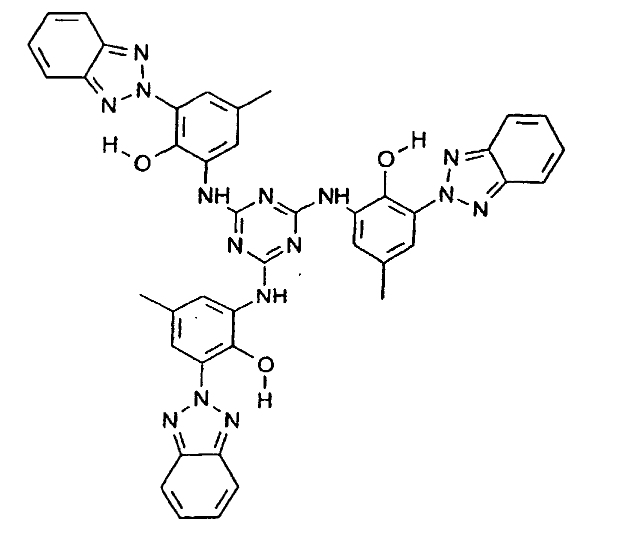

On chauffe au reflux pendant 12 heures sous azote un mélange de 2-(2-hydroxy-3-amino-5-methylphenyl)benzotriazole (1.2 g. 5 10⁻³ mole) et le chlorure de cyanuryle (0.276 g, 1.5 10⁻³ mole) dans 50 ml de xylène. Un précipité s'est formé que l'on filtre, rince au xylène et sèche sous vide. On obtient ainsi le dérivé (1 g, Rendement = 83%) présentant les caractéristiques suivantes :
- poudre jaune pâle
- Pf > 270 °C.

| Analyse élémentaire pour C₄₂H₃₃N₁₅O_{3 :} | | | |
|---|---|---|---|
| théorie | C : 63.39 | H : 4.18 | N : 26.40 |
| trouvé | C : 64.09 | H : 4.25 | N : 26.12 |

Cette 2,4,6-tris[(3'-benzotriazol-2-yl-2'-hydroxy-5'-methyl) phenylamino]-s-triazine a été tamisée à 50 µm sur un tamis de monture Inox-toile Inox. Puis elle a été dispersée dans de la vaseline blanche vendue sous la dénomination commerciale « Codex 236 » par la société Sarega à la température de fusion commerciale « Codex 236 » par la société Sarega à la température de fusion de la vaseline et à raison de 5 g de 2,4,6-tris[(3'-benzotriazol-2-yl-2'-hydroxy-5'-methyl) phenylamino]-s-triazine tamisée pour 100 g de vaseline. Ce mélange a ensuite été traité aux ultra-sons pour assurer une dispersion homogène. Un film d'une épaisseur de 10 µm a été analysé.

Le spectre d'absorption UV solide a été obtenu à partir d'un spectrophotomètre Shimadzu UV 2101 PC.

On voit clairement que la 2,4,6-tris[(3'-benzotriazol-2-yl-2'-hydroxy-5'-methyl) phenylamino]-s-triazine absorbe dans l'ensemble du rayonnement UV (280-400 nm).

### EXEMPLE 2 :

### Préparation de la 2,4,6-tris[(3'-benzotriazol-2-yl-2'-hydroxy-5'-ter-octyl) phénylamino]-s-triazine :

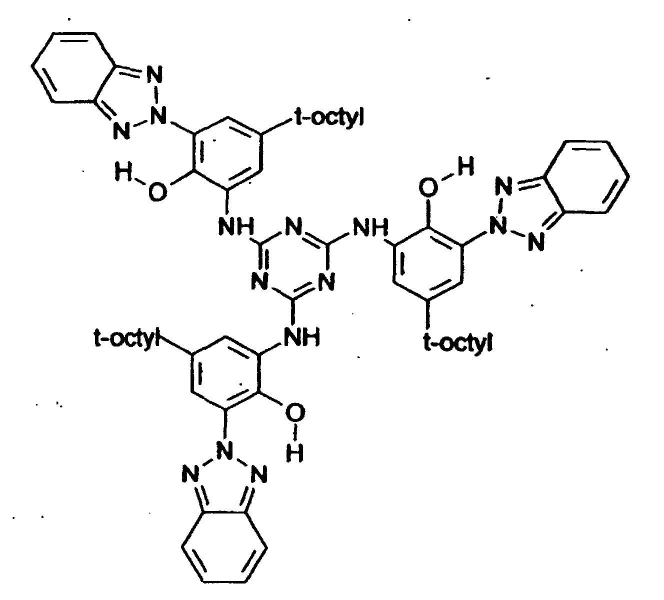

Dans un ballon tout équipé, on introduit le 2-(2-hydroxy-3-amino-5-ter-octylphenyl)benzotriazole (3,38 g, 0,01 mole) dans 50 ml de xylène. A température ambiante, sous courant d'azote, on introduit par portions le chlorure de cyanuryle (0,553 g, 3x10⁻³ mole) en 30 minutes, on chauffe le tout à 60 °C. On laisse le mélange à cette température pendant 1 heure puis on porte au reflux pendant 3 heures 30 minutes. Le mélange limpide est refroidi vers 60 °C ; on y ajoute 60 ml d'éthanol et le précipité obtenu est filtré, lavé à l'éthanol puis à l'eau. On obtient ainsi le dérivé (3,3 g, rendement 100%) ayant les caractéristiques suivantes :
- poudre jaune pâle
- Pf > 270°C
- UV (CHCl₃) λₘₐₓ=326 nm, εₘₐₓ=68 600
- UV : (sous forme solide) : voir figure 2 (spectre d'absorption)

| Analyse élémentaire pour C₆₃H₇₅N₁₅O₃: | | | |
|---|---|---|---|
| théorie | C : 69,40 | H : 6,93 | N : 19,27 |
| trouvé | C : 69,39 | H : 6,96 | N : 18,95 |

On a mesuré le spectre d'absorption UV solide de cette 2,4,6-tris[(3'-benzotriazol-2-yl-2'-hydroxy-5'-ter-octyl) phénylaminol-s-triazine selon le même protocole que dans l'exemple 1. On voit également clairement que la 2,4,6-tris[(3'-benzotriazol-2-yl-2'-hydroxy-5'-ter-octyl) phénylamino]-s-triazine absorbe dans l'ensemble du rayonnement UV (280-400 nm).

### EXEMPLE 3 :

On donne ci-après un exemple concret d'une composition cosmétique se présentant sous la forme d'une émulsion de type huile-dans-eau (les quantités sont exprimées en % de poids par rapport au poids total de la composition) :
- huile de silicone 1.5 %
- adipate de diisopropyle 15 %
- dérivé de l'exemple 1 5 %
- glycérine 20 %
- parfum, conservateurs qs
- eau qsp 100%

Cette composition a été réalisée de la manière suivante : après réalisation de l'émulsion, on a dispersé le filtre vers 40°C. On a ensuite homogénéisé la crème obtenue à la tricylindre.

Cette composition absorbe dans l'ensemble du rayonnement UV.

## Revendications

1. Composé de formule (I) suivante : 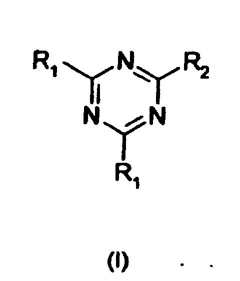 dans laquelle les symboles R₁, identiques ou différents, sont les radicaux de formules (II) ou (III) suivantes : 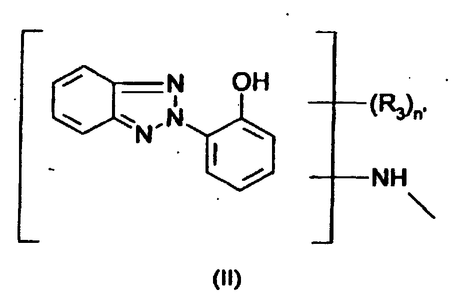 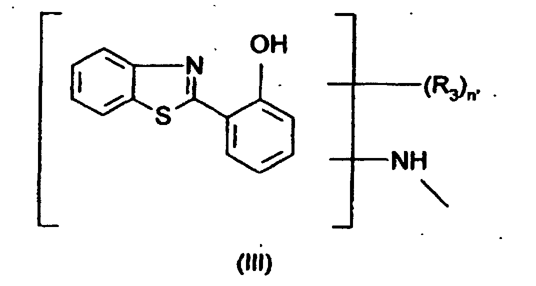
- R₂ est un halogène, N(R₄)₂, OR₅ ou un groupement R₁,
- R₃ ,identiques ou différents, sont des radicaux alkyle en C₁-C₈ linéaires ou ramifiés, des radicaux alkoxy en C₁-C₃ étant entendu que, dans ce dernier cas, deux R₃ adjacents d'un même noyau aromatique peuvent former ensemble un groupement alkylidène dioxy dans lequel le groupe alkylidène contient de 1 à 2 atomes de carbone, OH, NHCOCH₃ ou NH₂,
- R₄, identiques ou différents, désignent un atome d' hydrogène ou un radical alkyle en C₁-C₆ linéaire ou ramifié, deux R₄ pouvant former ensemble un cycle de 4 ou 5 atomes de carbone,
- n est 0, 1, 2, 3,
- R₅ est un hydrogène ou un radical alkyle en C₁-C₆.

2. Composé selon la revendication 1, **caractérisé par le fait que** les radicaux R₁ et le radical R₂ sont identiques.

3. Composé selon la revendication 2, **caractérisé par le fait que** lesdits radicaux R₁ et R₂ désignent un radical de formule (II).

4. Composé selon la revendication 3, **caractérisé par le fait qu'**il est choisi parmi les composés suivants : la 2,4,6-tris[(3'-benzotriazol-2-yl-2'-hydroxy-5'-methyl) phenylamino]-s-triazine et 2,4,6-tris[(3'-benzotriazol-2-yl-2'-hydroxy-5'-ter-octyl) phénylaminol-s-triazine.

5. Procédé de préparation d'un composé de formule (I) tel que défini à l'une quelconque des revendications 1 à 4, **caractérisé par le fait qu'**il consiste à faire réagir un composé de formule (IV) ci-dessous avec les dérivés de formules R₁H et R₂H selon le schéma réactionnel ci-dessous : 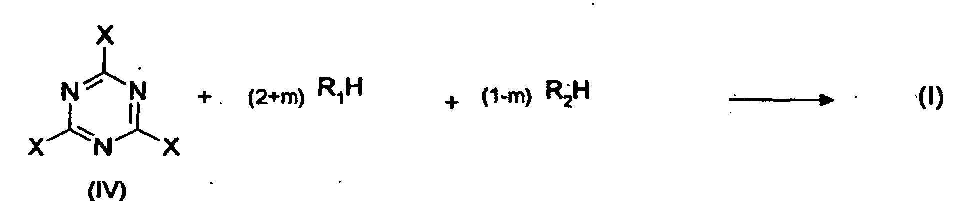 où :
- R₁ et R₂ répondent aux définitions de l'une quelconque des revendications 1 à 5,
- X représente un halogène, en particulier le chlore ou le brome,
- m est 0 ou 1.

6. Composition destinée à protéger la peau et/ou les cheveux contre le rayonnement ultraviolet, en particulier le rayonnement solaire, **caractérisée par le fait qu'**elle comprend une quantité efficace d'au moins un composé défini à l'une quelconque des revendications 1 à 4.

7. Composition destinée à protéger un verre organique et/ou minéral ou une matière plastique contre le rayonnement ultraviolet, en particulier le rayonnement solaire, **caractérisée par le fait qu'**elle comprend une quantité efficace d'au moins un composé défini à l'une quelconque des revendications 1 à 4.

8. Composition selon la revendication 6 ou 7, **caractérisée par le fait que** ledit composé est sous forme de particules.

9. Composition selon la revendication 8, **caractérisée par le fait que** la taille moyenne desdites particules est inférieure à 20 µm.

10. Composition selon l'une quelconque des revendications 6 à 9, **caractérisée par le fait qu'**elle contient de 0,1 à 15 % en poids, par rapport au poids total de la composition, dudit composé.

11. Composition selon l'une quelconque des revendications 7 à 10, **caractérisée par le fait qu'**il s'agit d'un vernis destiné à protéger un verre organique et/ou minéral ou une matière plastique.

12. Utilisation d'au moins un composé défini à l'une quelconque des revendications 1 à 4 dans des, ou pour la fabrication de, compositions destinées à protéger la peau et/ou les cheveux contre le rayonnement ultraviolet, en particulier au rayonnement solaire.

13. Utilisation d'au moins un composé défini à l'une quelconque des revendications 1 à 4 dans des, ou pour la fabrication de vernis pour la protection des verres organiques et/ou minéraux ou des matières plastiques contre le rayonnement ultraviolet, en particulier contre le rayonnement solaire.

14. Procédé cosmétique de protection de la peau et/ou les cheveux contre le rayonnement ultraviolet, en particulier le rayonnement solaire , **caractérisé par le fait qu'**il consiste à appliquer sur la peau et/ou les cheveux une quantité efficace d'une composition définie à l'une quelconque des revendications 6 et 8 à 10.

15. Procédé de protection d'une matière plastique contre le rayonnement ultraviolet, en particulier contre le rayonnement solaire, **caractérisé par le fait qu'**il consiste à incorporer dans une matière plastique une quantité efficace d'un composé tel que défini à l'une quelconque des revendications 1 à 4 ou d'une composition telle que définie dans définie à l'une quelconque des revendications 7 à 11.

16. Procédé de protection d'un verre minéral ou organique contre le rayonnement ultraviolet, en particulier contre le rayonnement solaire , **caractérisé par le fait qu'**il consiste à appliquer une quantité efficace d'un composé tel que défini à l'une quelconque des revendications 1 à 4 ou d'une composition telle que définie dans la revendication 11, à la surface d'un verre minéral ou organique.

17. Une composition de matière plastique ou verrière protégée selon le procédé de la revendication 15 ou 16.

## Patentansprüche

1. Verbindungen der folgenden Formel (I): 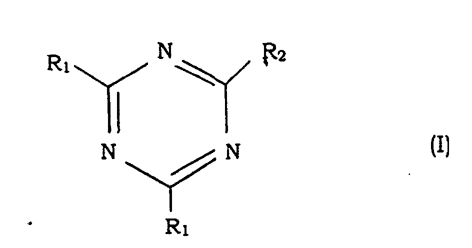 worin:
- die Gruppen R₁, die identisch oder voneinander verschieden sein können, unter den Gruppen der folgenden Formeln (II) oder (III) ausgewählt sind: 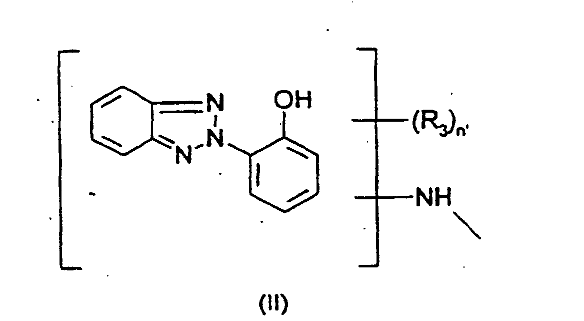 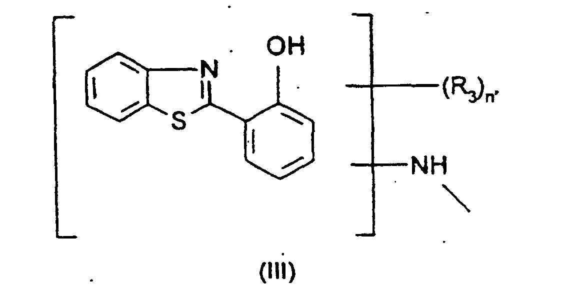
- R₂ ein Halogenatom, N(R₄)₂, OR₅ oder eine Gruppe R₁ bedeutet,
- die Gruppen R₃, die identisch oder voneinander verschieden sein können, bedeuten: geradkettige oder verzweigte C₁₋₈-Alkylgruppen oder C₁₋₃-Alkoxygruppen, mit der Maßgabe, dass im letzten Fall zwei angrenzende Gruppen R₃ an dem gleichen aromatischen Ring gemeinsam eine Alkylidendioxygruppe bilden können, worin die Alkylidengruppe 1 bis 2 Kohlenstoffatome aufweist, OH, NHCOOCH₃ oder NH₂,
- die Gruppen R₄, die identisch oder voneinander verschieden sein können, Wasserstoff oder geradkettige oder verzweigte C₁₋₆-Alkylgruppen bedeuten, wobei zwei Gruppen R₄ gemeinsam einen Ring mit 4 oder 5 Kohlenstoffatomen bilden können,
- n 0, 1, 2 oder 3 bedeutet, und
- die Gruppe R₅ Wasserstoff oder C₁₋₆-Alkyl bedeutet.

2. Verbindungen nach Anspruch 1, **dadurch gekennzeichnet, dass** die Gruppen R₁ und R₂ identisch sind.

3. Verbindungen nach Anspruch 2, **dadurch gekennzeichnet, dass** die Gruppen R₁ und R₂ identisch sind und jeweils eine Gruppe der Formel (II) bedeuten.

4. Verbindungen nach Anspruch 3, **dadurch gekennzeichnet, dass** sie unter den folgenden Verbindungen ausgewählt sind:
- 2,4,6-Tris((3'-benzotriazol-2-yl-2'-hydroxy-5'-methyl)-phenylamino)-s-triazin, und
- 2,4,6-Tris((3'-benzotriazol-2-yl-2'-hydroxy-5'-*t*-octyl)-phenylamino)-s-triazin.

5. Verfahren zur Herstellung einer Verbindung der Formel (I) nach einem der Ansprüche 1 bis 4, **dadurch gekennzeichnet, dass** es darin besteht, die folgende Verbindung der Formel (IV) nach dem folgenden Reaktionsschema mit den Derivaten der Formeln R₁H und R₂ H umzusetzen: 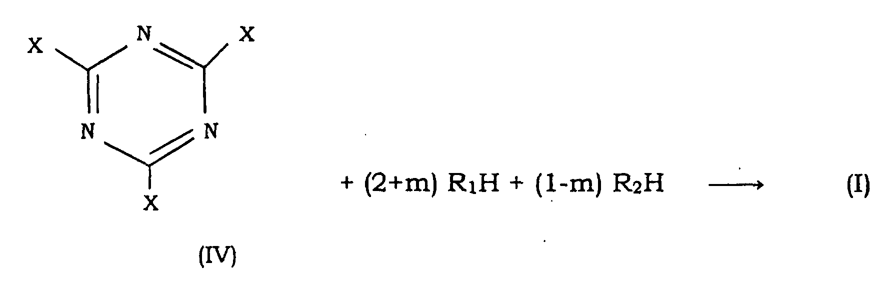 worin:
- R₁ und R₂ den Definitionen der Ansprüche 1 bis 5 entsprechen,
- X Halogen und insbesondere Chlor oder Brom bedeutet, und
- m 0 oder 1 ist.

6. Zusammensetzung zum Schutz der Haut und/oder der Haare gegen UV-Strahlung und insbesondere Sonnenlicht, **dadurch gekennzeichnet, dass** sie eine wirksame Menge mindestens einer Verbindung nach einem der Ansprüche 1 bis 4 enthält.

7. Zusammensetzung zum Schutz von organischen und/oder anorganischen Gläsern oder Kunststoffen gegen UV-Strahlung und insbesondere Sonnenlicht, **dadurch gekennzeichnet, dass** sie eine wirksame Menge mindestens einer Verbindung nach einem der Ansprüche 1 bis 4 enthält.

8. Zusammensetzung nach Anspruch 6 oder 7, **dadurch gekennzeichnet, dass** die Verbindung in Partikelform vorliegt.

9. Zusammensetzung nach Anspruch 8, **dadurch gekennzeichnet, dass** die mittlere Größe der Partikel unter 20 µm liegt.

10. Zusammensetzung nach einem der Ansprüche 6 bis 9, **dadurch gekennzeichnet, dass** sie 0,1 bis 15 Gew.-% der Verbindung, bezogen auf das Gesamtgewicht der Zusammensetzung, enthält.

11. Zusammensetzung nach einem der Ansprüche 7 bis 10, **dadurch gekennzeichnet, dass** es sich um einen Lack zum Schutz von organischen und/oder anorganischen Gläsern oder Kunststoffen handelt.

12. Verwendung mindestens einer Verbindung nach einem der Ansprüche 1 bis 4 in Zusammensetzungen zum Schutz der Haut und/oder der Haare gegen UV-Strahlung und insbesondere gegenüber Sonnenlicht oder zur Herstellung dieser Zusammensetzungen.

13. Verwendung mindestens einer Verbindung nach einem der Ansprüche 1 bis 4 in oder zur Herstellung von Lacken zum Schutz von organischen und/oder anorganischen Gläsern oder Kunststoffen gegen UV-Strahlung und insbesondere gegenüber Sonnenlicht.

14. Kosmetisches Verfahren zum Schutz der Haut und/oder der Haare gegen UV-Strahlung und insbesondere gegenüber Sonnenlicht, **dadurch gekennzeichnet, dass** es darin besteht, eine wirksame Menge einer Zusammensetzung nach einem der Ansprüche 6 und 8 bis 10 auf die Haut und/oder der Haare aufzutragen.

15. Verfahren zum Schutz von Kunststoffen gegen UV-Strahlung und insbesondere gegenüber Sonnenlicht, **dadurch gekennzeichnet, dass** es darin besteht, in den Kunststoff eine wirksame Menge einer Verbindung nach einem der Ansprüche 1 bis 4 oder einer Zusammensetzung nach einem der Ansprüche 7 bis 11 einzuarbeiten.

16. Verfahren zum Schutz von organischen und/oder anorganischen Gläsern gegen UV-Strahlung und insbesondere gegenüber Sonnenlicht, **dadurch gekennzeichnet, dass** es darin besteht, eine wirksame Menge einer Verbindung nach einem der Ansprüche 1 bis 4 oder einer Zusammensetzung nach Anspruch 11 auf die Oberfläche eines organischen und/oder anorganischen Glases aufzubringen.

17. Kunststoffe oder Gläser, die durch das Verfahren nach einem der Ansprüche 15 oder 16 geschützt sind.

## Claims

1. Compound of formula (I) below: 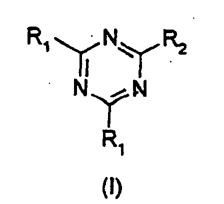 in which the symbols R₁, which may be identical or different, are radicals of formula (II) or (III) below: 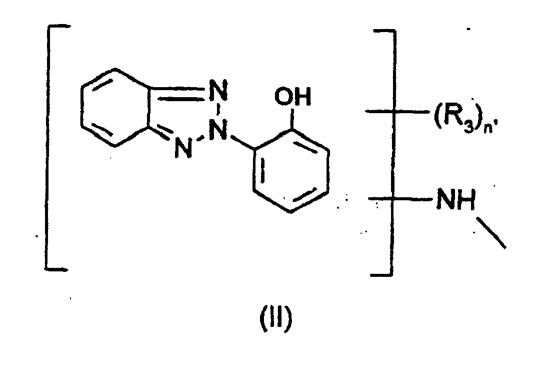 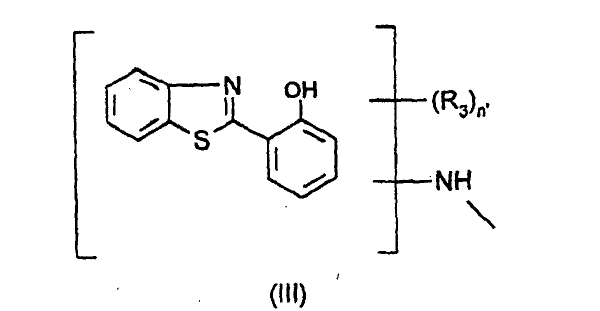
- R₂ is a halogen, N(R₄)₂, OR₅ or a group R₁,
- R₃, which may be identical or different, are linear or branched C₁-C₈ alkyl radicals, C₁-C₃ alkoxy radicals, it being understood that, in the latter case, two adjacent groups R₃ on the same aromatic ring can together form an alkylidenedioxy group in which the alkylidene group contains 1 or 2 carbon atoms, OH, NHCOCH₃ or NH₂,
- R₄, which may be identical or different, denote a hydrogen atom or a linear or branched C₁-C₆ alkyl radical, it being possible for two groups R₄ together to form a ring of 4 or 5 carbon atoms,
- n is 0, 1, 2 or 3,
- R₅ is a hydrogen or a C₁-C₆ alkyl radical.

2. Compound according to Claim 1, **characterized in that** the radicals R₁ and the radical R₂ are identical.

3. Compound according to Claim 2, **characterized in that** the said radicals R₁ and R₂ denote a radical of formula (II).

4. Compound according to Claim 3, **characterized in that** it is chosen from the following compounds: 2,4,6-tris[(3'-benzotriazol-2-yl-2'-hydroxy-5'-methyl)phenylamino]-s-triazine and 2,4,6-tris[(3'-benzotriazol-2-yl-2'-hydroxy-5'-tert-octyl)phenylamino]-s-triazine.

5. Process for preparing a compound of formula (I) as defined in any one of Claims 1 to 4, **characterized in that** it consists in reacting a compound of formula (IV) below with the derivatives of formulae R₁H and R₂H according to the reaction scheme below: 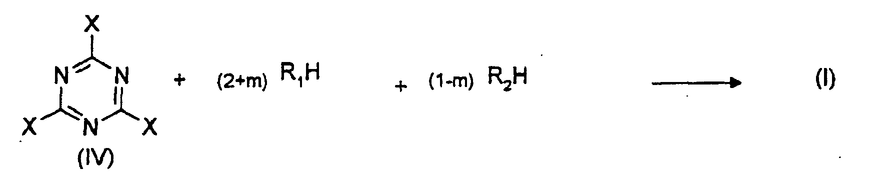 in which:
- R₁ and R₂ correspond to the definitions of any one of Claims 1 to 5,
- X represents a halogen, in particular chlorine or bromine,
- m is 0 or 1.

6. Composition intended for protecting the skin and/or the hair against ultraviolet radiation, in particular solar radiation, **characterized in that** it comprises an effective amount of at least one compound defined in any one of Claims 1 to 4.

7. Composition intended for protecting an organic and/or mineral glass or a plastic against ultraviolet radiation, in particular solar radiation, **characterized in that** it comprises an effective amount of at least one compound defined in any one of Claims 1 to 4.

8. Composition according to Claim 6 or 7, **characterized in that** the said compound is in the form of particles.

9. Composition according to Claim 8, **characterized in that** the average size of the said particles is less than 20 µm.

10. Composition according to any one of Claims 6 to 9, **characterized in that** it contains from 0.1 to 15% by weight, relative to the total weight of the composition, of the said compound.

11. Composition according to any one of Claims 7 to 10, **characterized in that** it is a varnish intended for protecting an organic and/or mineral glass or a plastic.

12. Use of at least one compound defined in any one of Claims 1 to 4, in, or for the manufacture of, compositions intended for protecting the skin and/or the hair against ultraviolet radiation, in particular solar radiation.

13. Use of at least one compound defined in any one of Claims 1 to 4 in, or for the manufacture of, varnishes for protecting organic and/or mineral glasses or plastics against ultraviolet radiation, in particular against solar radiation.

14. Cosmetic process for protecting the skin and/or the hair against ultraviolet radiation, in particular solar radiation, **characterized in that** it consists in applying an effective amount of a composition defined in any one of Claims 6 and 8 to 10 to the skin and/or the hair.

15. Process for protecting a plastic against ultraviolet radiation, in particular against solar radiation, **characterized in that** it consists in incorporating an effective amount of a compound as defined in any one of Claims 1 to 4 or of a composition as defined in any one of Claims 7 to 11 into a plastic.

16. Process for protecting a mineral or organic glass against ultraviolet radiation, in particular against solar radiation, **characterized in that** it consists in applying an effective amount of a compound as defined in any one of Claims 1 to 4 or of a composition as defined in Claim 11 to the surface of a mineral or organic glass.

17. Composition of plastic or glass material protected according to the process of Claim 15 or 16.
